# EUROPÄISCHE PATENTSCHRIFT

(11) **EP 2 563 116 B1**
(45) Veröffentlichungstag und Bekanntmachung des Hinweises auf die Patenterteilung: **12.11.2014**
(21) Anmeldenummer: 11708288.3
(22) Anmeldetag: 16.03.2011
(51) Int. Cl.: A01N 25/10, A01N 31/02, A01N 33/12, A01N 47/44, A01N 43/80, A01P 1/00, A61L 2/18, C11D 7/10, C11D 7/26, C11D 3/48, B08B 7/00

(54) **FORMBARE MASSE ZUR DESINFEKTION VON OBERFLÄCHEN AUF BASIS VON VERNETZTEN HYDROPROPYLIERTEN POLYGALACTOMANNAN**
MOLDABLE MATERIAL FOR DISINFECTING SURFACES BASED ON CROSSLINKED HYDROXYPROPYLATED POLYGALACTOMANNAN
MATÉRIAU MOULABLE POUR LA DÉSINFECTION DE SURFACES À BASE DE POLYGALACTOMANNAN HYDROXYPROPYLÉ RÉTICULÉ

(30) Priorität: 26.04.2010 CH 6002010
(43) Veröffentlichungstag der Anmeldung: 06.03.2013
(73) Patentinhaber: Joker AG, 3210 Kerzers/FR (CH)
(72) Erfinder: FLURY, Meinrad, 3210 Kerzers/FR (CH); DIETRICH, René, H., 8597 Landschlacht (CH)
(74) Vertreter: Schneider Feldmann AG Patent- und Markenanwälte
(86) Internationale Anmeldenummer: PCT/EP2011/053921
(87) Internationale Veröffentlichungsnummer: WO 2011/134715

(56) Entgegenhaltungen:
- CN-A- 101 233 844
- US-A- 4 543 131
- US-A- 5 804 213

## Beschreibung

### Technisches Gebiet

Die Erfindung betrifft Mittel zur Desinfektion von schwierig zu reinigenden Oberflächen.

### Stand der Technik

In Krankenhäusern sind auch in der heutigen Zeit bakterielle Infektionen ein grosses Problem und führen zu vielen Komplikationen. Zwar stehen schon länger Antibiotika zur Behandlung bakterieller Infektionskrankheiten zur Verfügung, jedoch haben sich speziell im Umfeld von Krankenhäusern Krankheitskeime entwickelt, die gegen verschiedene Antibiotika resistent sind. Solche multiresistente Bakterien stellen ein immer grösseres Problem dar insbesondere in der Intensivmedizin, da speziell die Patienten in Krankenhäusern oft ein geschwächtes Immunsystem aufweisen, und darum gegen Infektionen besonders anfällig sind. Sehr verbreitete antibiotikaresistente Stämme sind Methicillin-resistente Staphylococcus aureus (MRSA)-Stämme, bei denen auf Intensivstationen in den USA bereits eine Erkrankungshäufigkeit von mehr als 50% auf ein Jahr gerechnet auftritt.

Ähnliche Probleme bestehen auch in Tierarztpraxen, da auch Katzen und Hunde häufig Träger von MRSA sind. Studien haben gezeigt, dass auch in diesem Fall der unsachgemässe Umgang mit Antibiotika zu multiresistenten Keimen bei Haustieren führt («Occurrence of highly fluoroquinolone-resistant and methicillin-resistant Staphylococcus aureus in domestic animals», A.E. Lin, J.E. Davies, Can. J. Microbiol. 53, 925, (2007)). Diese werden dann auf die Halter und das Personal von Tierarztpraxen übertragen, von wo aus sie dann wieder auf Tiere oder Menschen weiter übertragen werden können («High risk for nasal carriage of methicillin-resistant Staphylococcus aureus among Danish veterinary practitioners», A. Moodley et al., Scand. J. Work, Envir. & Health 34, 151 (2008)).

Das effizienteste Mittel zur Vermeidung von Komplikationen durch Infekte ist bekanntermassen die Vermeidung der Infektion selber durch geeignete Hygienemassnahmen, insbesondere durch eine regelmässige und ausreichende Desinfektion von Gegenständen und Oberflächen mit denen ein Patient direkt oder indirekt in Kontakt kommen kann. Flache, glatte Oberflächen können mit herkömmlichen flüssigen Desinfektionsmitteln relativ problemlos desinfiziert werden. Verbrauchsgegenstände im Spitalbereich werden in der Regel bereits sterilisiert abgepackt zur Verfügung gestellt. Mehrmals verwendbare Vorrichtungen und Gegenstände wiederum, wie beispielsweise Operationsbesteck oder medizinische Instrumente können in Autoklaven sterilisiert werden.

Problematisch hingegen sind Alltagsgegenstände, wie beispielsweise Kugelschreiber, Mobiltelefone, Pager, Brillen, Computertastaturen, etc. deren Gebrauch auch in Krankenhäusern unvermeidbar ist. Solche Gegenstände kommen häufig in Kontakt mit den Händen, die naturgemäss besonders keimbelastet sind. Auf diese Weise können Keime durch Besucher und Patienten, aber insbesondere durch das Pflegepersonal selber, weiträumig verschleppt werden. Ein anderes Problem sind elektronische Geräte, die schwerzugängliche Ritzen beispielsweise bei Bedienungsknöpfen aufweisen können, die durch eine herkömmliche Oberflächendesinfektion nicht zugänglich sind und so als Keimherde dienen.

Die genannten Gegenstände sind in aller Regel nicht autoklavierbar, und flüssige Desinfektionsmittel sind nicht praktikabel, da sie bei oberflächlicher Anwendung nicht in alle Ritzen dringen. Flüssige Desinfektionsmittel können zudem elektronische Bauteile beschädigen. Es wäre deshalb wünschenswert, eine Möglichkeit zu haben, auch solche problematischen Gegenstände und Oberflächen einfach und effektiv desinfizieren zu können.

WO 02/055642 offenbart ein Reinigungsmittel zum Entfernen von Feststoffpartikeln von Oberflächen, bei welchem eine pseudoplastische Polygalactomannan-Gelmasse in enge Zwischenräume eindringt und vorhandene Feststoffpartikel aufnimmt. Die aufgenommenen Partikel werden in die Masse eingebunden, so dass laufend eine unverbrauchte Oberfläche für die Reinigung zur Verfügung steht. Die genannte Reinigungsmasse ist auf die effiziente Aufnahme von Staub und Fusseln ausgerichtet. Die Oberflächenbenetzung ist für einen Einsatz als Desinfektionsmittel ungenügend.

Aus der CN 101 233 844 ist eine wässrige Gelatine zur Reinigung und Sterilisierung von Oberflächen, die die folgenden Komponenten enthalten bekannt:
a) 65-98,8 Gew.% Wasser;
b) 1-22 Gew.% eines Leimungsmittels;
c) 0,1-5 Gew.% eines Geliermittels;
d) 0,1-7 Gew.% eines Fungizids;
e) 0-33,7 Gew.% eines Hilfsmittels.

Unter anderem werden als bevorzugte Komponente die folgenden genannt:
b) Leimungsmittel: Hydroxypropylierte Guargummi;
c) Geliermittel: Organobor-Verbindung;
d) Fungizid: Chlorhexedin oder quaternäre Ammoniumverbindungen;
e) Hilfsmittel: Äthanol, zur Verbesserung der Sterilisierungs- sowie der Kolloid-Eingeschaften.

Die Gelatine eignen sich zur Reinigung und Sterilisierung von groben Oberflächen und schwer zugänglichen Ritzen wie z.B. bei Bedienungsknöpfen von elektronischen Geräten.

### Aufgabe der Erfindung

Aufgabe der Erfindung ist es, ein Mittel zur Desinfektion von Oberflächen zur Verfügung zu stellen, das die oben erwähnten und andere Nachteile nicht aufweist. Insbesondere soll ein solches Desinfektionsmittel in der Lage sein, auch in schwer zugängliche Ritze einzudringen, um dort vorhandene Keime abzutöten, hierbei soll eine höhere Konzentration an

Desinfektionsmitteln in Form von niedermolekularen Alkoholen erzielbar sein, ohne unerwunschte Abnahme der Elastizität zu bewirken.

Ein solches Desinfektionsmittel soll Keime effektiv und schnell abtöten, um so die Hygieneanforderungen im Spitalbereich zu erfüllen und eine Verschleppung von Keimen effizient zu unterbinden. Insbesondere soll es die entsprechend festgelegten Normen erfüllen, insbesondere die Europäischen Normen EN 1040 und EN13697.

Diese und andere Aufgaben werden gelöst durch eine erfindungsgemässe formbare Masse zur Verwendung als Desinfektionsmittel gemäss dem unabhängigen Anspruch. Weitere vorteilhafte Ausführungsformen sind in den abhängigen Ansprüchen gegeben.

### Darstellung der Erfindung

Eine erfindungsgemässe formbare Masse zur Verwendung als Desinfektionsmittel umfasst 25 bis 40 Gewichtsprozent, bevorzugt 30 bis 35 Gewichtsprozent einer Lösungsmittelkomponente bestehend aus niedermolekularen, mit Wasser mischbaren Alkoholen, 1 bis 10 Gewichtsprozent, bevorzugt 2 bis 7 Gewichtsprozent einer bakteriziden Wirkkomponente, 3 bis 6 Gewichtsprozent hydroxypropyliertes Polygalactomannan mit einem Veretherungsgrad zwischen 0.3 und 1.5, und 0.1 bis 0.8 Gewichtsprozent Borsäure oder eine äquivalente Menge Bor-Ionen, bei einem pH-Wert zwischen 6 und 10. Die restlichen Gewichtsanteile sind vorzugsweise ergänzt mit Wasser.

Vorteilhaft ist eine erfindungsgemässe Masse dazu geeignet, Kleinpartikel, insbesondere biogene Verunreinigungen, auf ihrer Oberfläche haftend aufzunehmen. Dies hat den Vorteil, dass neben der sofortigen lokalen Desinfektionswirkung einer erfindungsgemässen Masse Kleinpartikel wie eben zum Beispiel Keime auf der Oberfläche haften bleiben. Dort werden die Keime ebenfalls abgetötet, wobei die Verweilzeit wesentlich höher ist als auf der zu reinigenden Oberfläche, und damit die bakterizide Wirkung absolut. Durch Kneten und Formen der Masse, wie es bei normaler Anwendung der erfindungsgemässen Masse automatisch geschieht, gelangen die Partikel dauerhaft ins Innere der Masse, und die erneuerte Oberfläche kann weitere Fremdpartikel biogener und nicht-biogener Art aufnehmen.

In einer vorteilhaften Ausführungsform einer solchen erfindungsgemässen Masse ist die Lösungsmittelkomponente ausgewählt aus einer Gruppe bestehend aus Ethanol, 2-Propanol, Methanol, 1-Propanol, und Gemischen davon. In einer noch vorteilhafteren Variante ist die Lösungsmittelkomponente Ethanol, 2-Propanol oder ein Gemisch davon.

Als bakterizide Wirkkomponente einer erfindungsgemässen Masse wird vorteilhafterweise Benzalkoniumchlorid, Benzalkoniumsaccharinat, Didecyl-Dimethyl-Ammoniumchlorid, Chlorhexidin oder ein Gemisch davon verwendet.

In einer anderen vorteilhaften Variante einer erfindungsgemässen Masse beträgt der Veretherungsgrad des hydroxypropylierten Polygalactomannans zwischen 0.5 und 1.5. Besonders vorteilhaft ist ein Veretherungsgrad zwischen 0.6 und 1.2.

Die Wirkung eines erfindungsgemässen formbaren Desinfektionsmittels beruht darauf, dass diese statt als flüssiges Desinfektionsmittel als Gelmasse mit pseudoplastischem Fliessverhalten ausgestaltet ist. Wird ein erfindungsgemässes Desinfektionsmittel auf einen Gebrauchsgegenstand aufgedrückt, beispielsweise ein Mobiltelefon oder eine Computertastatur, dann kriecht die Masse auch in Ritzen und Spalten. Auf diese Art und Weise kommen auch sonst nicht zugängliche Oberflächen in Kontakt mit dem Desinfektionsmittel, so dass Keime abgetötet werden. Nach der gewünschten Wirkzeit, beispielsweise einer Minute, wird die Gelmasse vollständig vom Gegenstand abgezogen.

Eine vorteilhafte erfindungsgemässe Masse umfasst 25 bis 40 Gew.-, bevorzugt 30 bis 35 Gew.% Ethanol oder Isopropanol oder ein Gemisch davon. Ein hoher Gehalt an Ethanol/Isopropanol ist neben der desinfizierenden Wirkung insbesondere vorteilhaft, um eine ausreichende Oberflächenbenetzung der zu desinfizierenden Oberflächen sicherzustellen, damit auch bei kurzer Wirkzeit die gewünschte Desinfektionswirkung erhalten wird. Weitere geeignete Alkoholkomponenten sind andere niedermolekulare, mit Wasser mischbare Alkohole, wie beispielsweise Methanol oder 1-Propanol.

Zur Bildung der gelförmigen Grundmasse des formbaren Desinfektionsmittels umfasst dieses modifizierte, borvernetzte Polygalactomannane. Polygalactomannan, ein Polysaccharid, findet als Hydrokolloid Verwendung als Verdickungs- und Geliermittel, beispielsweise in der Nahrungsmitteltechnologie. Unmodifizierte Polygalactomannane sind in Ethanol nicht löslich.

Es hat sich gezeigt, dass bei gelförmigen Reinigungsmitteln, wie sie aus dem Stand der Technik bekannt sind, eine Erhöhung des Gehalts an Lösungsmitteln, wie er für ein erfindungsgemässes Desinfektionsmittel notwendig ist, zu einer ungenügenden Konsistenz des Gels führt. Je nach Borgehalt und pH-Wert kann eine weiche, klebrige oder eine spröde, bröckelnde Konsistenz resultieren. Ein solches Gel hat nicht mehr die notwendige pseudoplastische Fliessfähigkeit, welche notwendig ist, auch feine Ritzen und Spalten schnell zu hintergreifen und die dortigen Oberflächen zu desinfizieren.

Es wurde nun gefunden, dass die Verwendung von ausreichend hydroxypropyliertem Polygalactomannan die Toleranz des Gels für höhere Alkoholgehalte verbessert. Zu diesem Zweck muss der Veretherungsgrad des Polygalactomannans (Anzahl Hydroxypropylgruppen pro Anhydrohexoseeinheit des Polygalactomannans) mindestens 0.3 betragen, bevorzugt 0.5 bis 1.5, und besonders bevorzugt 0.6 bis 1.2. Versuche haben gezeigt, dass ein zu hoher Veretherungsgrad wiederum zu einer unerwünschten Abnahme der Elastizität der Gelmasse führt.

Eine erfindungsgemässe formbare Masse zur Verwendung als Desinfektionsmittel gemäss einer der bevorzugten Ausführungsformen umfasst 3-6 Gew.% Polygalactomannan, sowie 0.1-0.8 Gew.% Borsäure, bei einem pH-Wert zwischen 6 und 10. Zur Vernetzung eignen sich als Bor-Komponente beispielsweise Natriumtetraborat oder Borsäure. Der pH-Wert kann bei Bedarf mittels Zusatz von Phosphaten, beispielsweise Trinatriumphosphat, oder Natriumcarbonat eingestellt werden. Der Vernetzungsgrad muss zum einen so hoch eingestellt werden, dass die Verdampfungsrate des Alkohols nicht zu hoch ist, um die Lagerfähigkeit des Desinfektionsmittels zu gewährleisten, und zum anderen so niedrig sein, dass eine ausreichende Oberflächenbenetzung stattfindet.

Die Viskosität einer solchen erfindungsgemässen Masse, gemessen bei 25 °C, beträgt 50'000 bis 250'000 mPa.s, vorzugsweise zwischen 100'000 und 200'000 und am besonders bevorzugt zwischen 120'000 und 180'000 mPa.s.

Als primäre bakterizide und fungizide Wirkkomponente eines erfindungsgemässen formbaren Desinfektionsmittels sind prinzipiell Substanzen geeignet, welche in Wasser und niedermolekularen Alkoholen löslich sind, über einen weiten ph-Bereich Wirkung zeigen, hydrolysestabil sind und vorteilhaft mit Tensiden verträglich sind. Die entsprechende Substanz sollte für Mensch und Tier im Wesentlichen unschädlich sein. Es sollte die zu reinigenden Oberflächen nicht angreifen, insbesondere Kunststoffe und andere Polymermaterialien. Vorzugsweise ist die Substanz biologisch abbaubar. Geeignete Desinfektions-Wirkkomponenten sind beispielsweise quartäre Ammoniumverbindungen, wie beispielsweise Benzalkoniumchlorid (Alkyl-dimethyl-benzylammoniumchlorid CAS 68391-01-5), insbesondere mit C12 = 55%, C14 = 25%, C16 = 11%, C18 = 9%, Benzalkoniumsaccharinat, Didecyl-Dimethyl-Ammoniumchlorid (CAS 7173-51-5) oder dergleichen. Ebenfalls geeignet ist Chlorhexidin, beispielsweise als Chlorhexidin-Gluconat. Desinfizierend wirkende Aldehyde wie Formaldehyd und 1,5-Pentandial sind ebenfalls wirksam, aber aufgrund ihrer Giftigkeit nur für spezielle Anwendungsbereiche brauchbar, wo eine sporozide Wirkung gewünscht ist. Der Anteil an Desinfektionswirkkomponente beträgt zwischen 1 und 10 Gew.%, vorzugsweise zwischen 2 bis 7 Gew.%.

Ein erfindungsgemässe Masse kann zusätzlich wasserlösliche, hydrolysestabile Tenside enthalten, zur weiteren Verbesserung der Oberflächenbenetzung, beispielsweise nichtionische Tenside wie beispielsweise Polydimethylsiloxanpolyoxyalkylen-Copolymere.

Die restlichen Gewichtsanteile eines erfindungsgemässen Desinfektionsmittels neben den beschriebenen Inhaltsstoffen werden vorzugsweise mit Wasser ergänzt.

Ein erfindungsgemässes Desinfektionsmittel wurde mit genormten Tests auf seine bakterizide Wirkung insbesondere auf MRSA (ATTC 43300) getestet, gemäss Norm EN 1040 *(«Chemical disinfectants and antiseptics - Basic bactericidal activity test method and requirements (phase 1), 1997*».) und EN 1 3 6 9 7 *(»Chemical disinfectants and antiseptics* - *Quantitative non-porous surface test for the evaluation of bactericidal and*/*or fungicidal activity of chemical disinfectants used in food, industrial, domestic and institutional areas - test method and requirements without mechanical action (phase 2*/ *step 2)*»).

Die Tests ergaben sowohl für 1 Minute wie auch für 5 Minuten Wirkzeit die geforderte Reduktion der (MRSA)-Keimzahl um mehr als 100'000, was die Anforderungen der genannten Normen erfüllt.

## Patentansprüche

1. Formbare Masse zur Verwendung als Desinfektionsmittel, umfassend Wasser sowie
25-40 Ges.%, einer Lösungsmittelkomponente bestehend aus niedermolekularen, mit Wasser mischbaren Alkoholen;
1-10 Gew.%, einer bakteriziden Wirkkomponente;
3-6 Gew.% hydroxypropyliertes Polygalactomannan mit einem Veretherungsgrad von 0.3-1.5; und
0.1-0.8 Gew.% Borsäure oder eine dazu äquivalente Menge Bor-Ionen, bei einem pH-Wert zwischen 6 und 10.

2. Masse nach Anspruch 1, **dadurch gekennzeichnet, dass** die Masse dazu geeignet ist, Kleinpartikel, insbesondere biogene Verunreinigungen, auf ihrer Oberfläche haftend aufzunehmen.

3. Masse nach einem der vorhergehenden Ansprüche, **dadurch gekennzeichnet, dass** die Lösungsmittelkomponente ausgewählt ist aus einer Gruppe bestehend aus Ethanol, 2-Propanol, Methanol, 1-Propanol, und Gemischen davon.

4. Masse nach einem der vorhergehenden Ansprüche, **dadurch gekennzeichnet, dass** die bakteriziden Wirkkomponente ausgewählt ist aus einer Gruppe bestehend aus Benzalkoniumchlorid, Benzalkoniumsaccharinat, Didecyl-Dimethyl-Ammoniumchlorid, Chlorhexidin, und Gemischen davon.

5. Masse nach einem der vorangehenden Ansprüche, **dadurch gekennzeichnet, dass** der Veretherungsgrad des hydroxypropylierten Polygalactomannans 0,6-1,2 beträgt.

6. Masse nach Anspruch 1, **dadurch gekennzeichnet, dass** diese 30-40 Gew.% Lösungsmittelkomponente aus niedermolekularen mit Wassermischbaren Alkohole umfasst.

7. Masse nach Anspruch 1, **dadurch gekennzeichnet, dass** diese 2-7 Gew% einer bakteriziden Wirkkomponente umfasst.

8. Masse nach Anspruch 3, **dadurch gekennzeichnet, dass** diese Losungsmittelkomponente umgewählt ist aus einer Gruppe bestehend aus Ethanol, 2 - Propanol und Gemischen davon.

## Claims

1. A malleable compound for use as a disinfectant, comprising water and also
25 - 40 % by weight of a solvent component consisting of low-molecular alcohols that can be mixed with water;
1 - 10 % by weight of an active bactericidal component;
3 - 6 % by weight of hydroxypropylated polygalactomannan with a degree of etherification of 0.3 - 1.5; and
0.1 - 0.8 % by weight of boric acid or an equivalent amount of boron ions, at a pH value between 6 and 10.

2. The compound according to claim 1, **characterised in that** the compound is suitable for gathering small particles, in particular biogenic impurities, on its surface by adhesion.

3. The compound according to one of the preceding claims, **characterised in that** the solvent component is selected from the group consisting of ethanol, 2-propanol, methanol, 1-propanol, and mixtures thereof.

4. The compound according to one of the preceding claims, **characterised in that** the active bactericidal component is selected from a group consisting of benzalkonium chloride, benzalkonium saccharinate, dodecyl-dimethyl ammonium chloride, chlorhexidine, and mixtures thereof.

5. The compound according to one of the preceding claims, **characterised in that** the degree of etherification of the hydroxypropylated polygalactomannan is 0.6 - 1.2.

6. The compound according to claim 1, **characterised in that** it contains 30 - 40 % by weight of solvent component from low-molecular alcohols that can be mixed with water.

7. The compound according to claim 1, **characterised in that** it contains 2 - 7 % by weight of an active bactericidal component.

8. The compound according to claim 3, **characterised in that** the solvent component is selected from a group consisting of ethanol, 2-propanol, and mixtures thereof.

## Revendications

1. Pâte façonnable destinée à être utilisée comme produit désinfectant, comprenant de l'eau ainsi que
de 25 - 40 % en poids d'un composant solvant constitué d'alcools à faible poids moléculaire, pouvant être mélangés à de l'eau ;
de 1 - 10 % en poids d'un composant actif bactéricide ;
de 3 - 6 % en poids de polygalactomannane hydroxypropylé avec un degré d'éthérification de 0,3 - 1,5 ; et
de 0,1 - 0,8 % en poids d'acide borique ou une quantité équivalente d'ions de bore, pour une valeur pH comprise entre 6 et 10.

2. Pâte selon la revendication 1, **caractérisée en ce que** la pâte est adaptée pour absorber des petites particules, notamment des impuretés biogènes adhérant sur sa surface.

3. Pâte selon l'une quelconque des revendications précédentes, **caractérisée en ce que** le composant solvant est choisi dans un groupe comprenant l'éthanol, le 2-propanol, le méthanol, le 1-propanol et des mélanges de ces derniers.

4. Pâte selon l'une quelconque des revendications précédentes, **caractérisée en ce que** le composant actif bactéricide est choisi dans un groupe comprenant le chlorure de benzalconium, le saccharinate de benzalconium, le chlorure de didécyl-diméthyl-ammonium, la chlorhéxidine et des mélanges de ces derniers.

5. Pâte selon l'une quelconque des revendications précédentes, **caractérisée en ce que** le degré d'éthérisation du polygalactomannane hydroxypropylé est de 0,6 - 1,2.

6. Pâte selon la revendication 1, **caractérisée en ce qu'**elle comprend de 30 - 40 % en poids de composant solvant d'alcools à faible poids moléculaire pouvant être mélangés à de l'eau.

7. Pâte selon la revendication 1, **caractérisée en ce qu'**elle comprend de 2 - 7 % en poids d'un composant actif bactéricide.

8. Pâte selon la revendication 3, **caractérisée en ce que** ledit composant solvant est choisi dans un groupe comprenant l'éthanol, le 2-propanol et des mélanges de ces derniers.
